# EUROPEAN PATENT APPLICATION

(11) **EP 1 449 480 A1**
(43) Date of publication of application: **25.08.2004**
(21) Application number: 03256437.9
(22) Date of filing: 13.10.2003
(51) Int. Cl.: A61B 6/04

(54) **Improved indexing system for radiation therapy**

(30) Priority: 19.02.2003 US 368923
(71) Applicant: Med-Tec Iowa, Inc., Orange City, IA 51041 (US)
(72) Inventor: Korver, Clayton P., Orange City, Iowa 51041 (US); Hursh, Vince S., Orange City, Iowa 51041 (US)
(74) Representative: Thomson, Paul Anthony

(57) **Abstract**

A patient positioning device includes a table for supporting the patient, and having opposite sides with male indexing nodules (20) extending outwardly along each side edge of the table. A lock bar (22) extends across the table and has opposite ends with a female recess to retentively engage opposing pairs of nodules on the side edges of the table. A patient restraint member (32) mates with the lock bar to provide for repeatable and accurate positioning of the patient on the treatment table.

## Description

### BACKGROUND OF THE INVENTION

Patient positioning systems are used for accurate and reproducible positioning of a patient for radiation therapy, diagnostic imaging, surgery, and other medical procedures. During these procedures, it is important to immobilize a part or parts of the patient's body. Accurate positioning of the body part is also important in repeat treatments so that the precise same location of the bodies are exposed to the radiation each time. Therefore, different types of devices have been made to immobilize body parts and to index the body to the treatment table to assure proper and repeatable alignment for radiation therapy.

One example of such a patient positioning system is the Exact Indexed' Immobilization system sold by applicant, and described in U.S. Pat. No. 5,806,116. The Exact system utilizes a tabletop with indentations along opposite sides, and a lock bar extending across the tabletop with a disk at each end adapted to be received in opposing indentations. The Exact lock bar is secured to the tabletop with an eccentric cam which tightens the ends of the bar into engagement with the indentations of the tabletop.

The tables used in conventional patient positioning systems, such as the Exact system, are a solid foam core covered with a carbon fiber skin. In the Exact table, solid material must be imbedded into the opposite sides of the table into which the indexing notches are machined. Absent the solid material, the foam core would not have sufficient strength, if notched, to retentively engage the disks on the ends of the lock bar. Thus, the manufacturing process of the Exact table is somewhat.

While the Exact system is generally an acceptable product, there are features causing potential concern. For example, the rotation of the cam lever causes the bar to move slightly relative to the tabletop, such that the patient positioning is not precise and consistent. Also, the Exact lock bar utilizes an O-ring on each end of the bar, which is not resistant to radiation. Therefore, over time, the O-ring may degrade to a degree wherein the bar does not mount as securely to the tabletop. The O-ring may also ultimately fail due to repeated and extensive exposure to the radiation. Furthermore, the thermoplastic patient fixation or restraint devices normally used in patient positioning systems tend to shrink over time, as a result of the initial heating and molding of the thermoplastic. Such shrinkage ultimately may lead to bending of the Exact bar when the restraint is forced downwardly over the patient. Such bending of the bar may lead to migration of the bar out of the tabletop notches. Also, patient swelling may impart stresses on the Exact bar which may preclude secure attachment of the bar to the tabletop or Exact repeatable positioning of the bar.

Also, the solid material on the edges of the Exact tabletop increases attenuation due to the increased density of the material, relative to the foam core of the tabletop.

Therefore, a primary objective of the present invention is the provision of an improved patient positioning system for radiation therapy treatment.

A further objective of the present invention is the provision of an improved patient positioning system having a simplified manufacturing process.

Another objective of the present invention is the provision of an improved patient positioning system having male nodules on the edges of the tabletop and female recess on the ends of the lock bar.

Still another objective of the present invention is the provision of an improved patient positioning system having reduced attenuation.

Another objective of the present invention is the provision of a patient positioning system which is repeatably precise and accurate.

Another objective of the present invention is the provision of a patient positioning system which provides secure interlocking connection between the lock bar and the tabletop.

A further objective of the present invention is the provision of a patient positioning system which is easy and simple to use.

Another objective of the present invention is the provision of a patient positioning system which is durable in use and economical to manufacture.

These and other objectives have become apparent from the following description of the invention.

### BRIEF SUMMARY OF THE INVENTION

The patient positioning device of the present invention includes a table having opposite sides with male nodules spaced along each side. A patient restraint member is registered on an elongated lock bar and is adapted to extend over a portion of the patient's body to position the patient on the table. The lock bar has a female recess on each end which is adapted to snap fit onto opposing pairs of the nodules on each side of the table. The nodules each have a radius to matingly engage the recesses, which are self-centering on the nodules. The recesses retentively engage the nodules to prevent relative movement between the lock bar and the tabletop, thereby accurately positioning the patient for treatment.

### BRIEF DESCRIPTION OF THE DRAWINGS

Figure 1 is an exploded perspective view of the radiation therapy patient positioning system of the present invention, with a lock bar positioned above the table top and a patient restraint device before molding.

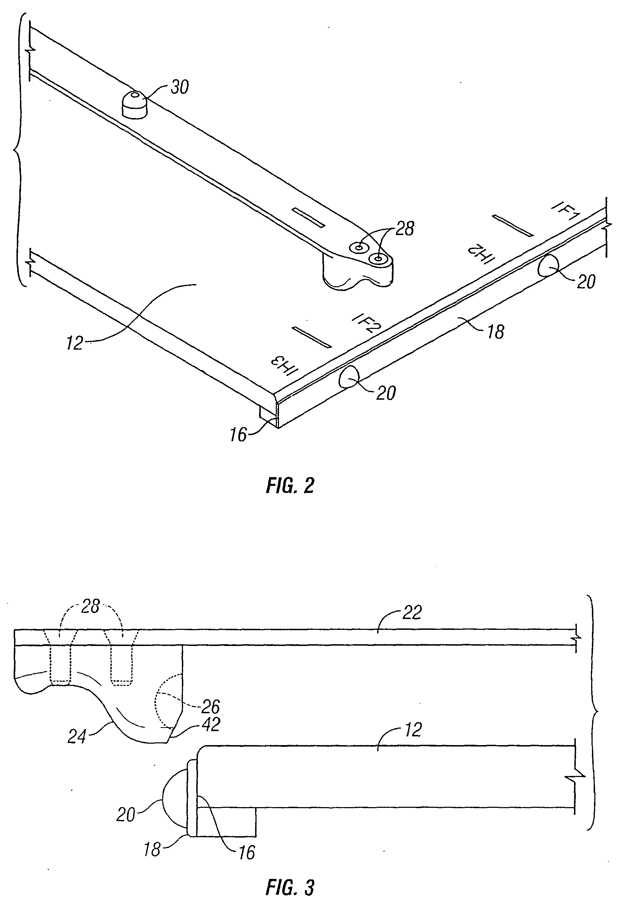

Figure 2 is an enlarged perspective view of one end of the lock bar and one edge of the table top.
Figure 3 is an exploded side elevation view of one end of the lock bar and table top edge.
Figure 4 is a side elevation view showing the lock bar with a molded patient restraint member.
Figure 5 is a perspective view showing an alternative embodiment.

### DETAILED DESCRIPTION OF THE DRAWINGS

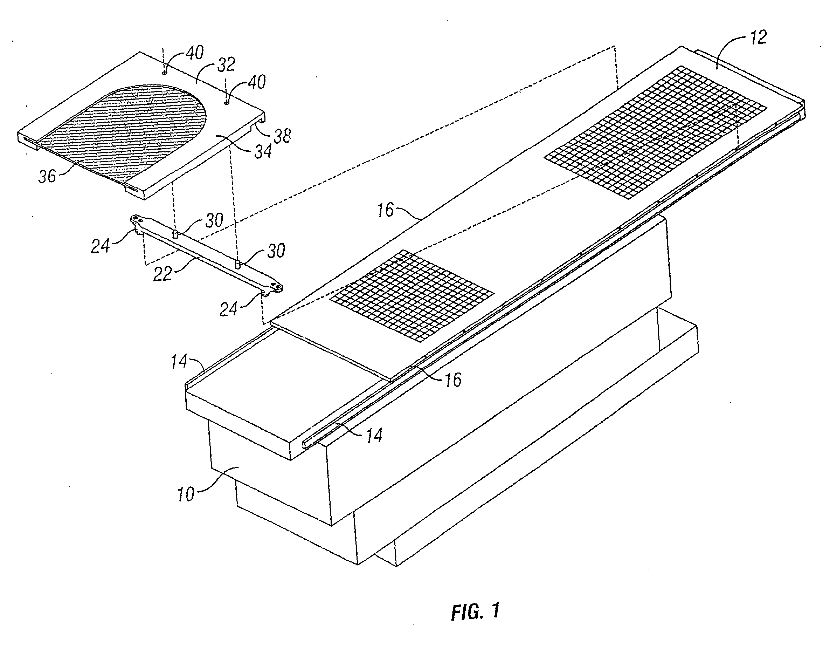

The patient restraint system of the present invention is particularly useful for accurate and repeatable patient positioning for radiation therapy treatment, as well as for other diagnostic and treatment procedures. As seen in Figure 1, a table base or carriage 10 as provided with a table top 12. The table top 12 is mounted for longitudinal movement along side rails 14 attached to the opposite sides of carriage 10.

The table top 12 has opposite side edges 16. A strip 18 having a plurality of spaced apart nodules or male projections. The male nodules 20 serve as indexing points for repeated treatment of individual patients. While the nodules 20 are shown in the drawings as being spherical in shape, it is understood that the nodules may have other shapes, particularly geometric cross sectional shapes such as triangles or trapezoids.

A lock bar 22 is adapted to attach to the table top 12. More particularly, the lock bar 22 includes opposite end members 24 and extend downwardly over the opposite side edges 16 of the table top. Each end member 24 has a female recess 26 which matingly and retentively engages the male nodules 20 with a snap fit. The male nodules 20 and the female recesses 26 have mating shapes. The end members 24 are secured to the lock bar 22 in any convenient manner, such as by screws 28.

The lock bar 22 also includes a pair of upwardly extending studs or shafts 30, which are mounted on the lock bar 22 in any convenient manner, such as by screws (not shown).

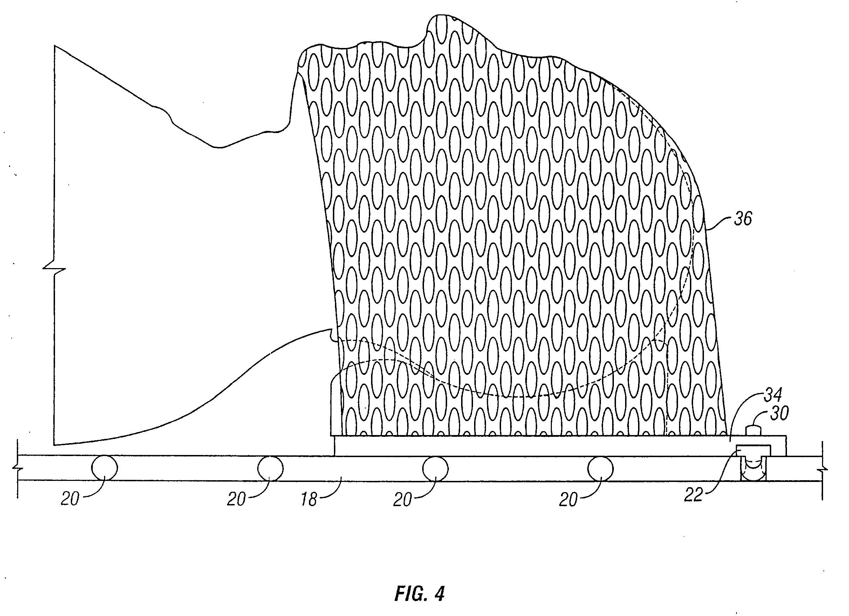

A patient restraint member 32 includes a frame 34 and a deformable membrane 26 adapted to be molded to the shape of the persons body part being restrained, such as a mask fitting over a patient's head, as shown in Figure 4. The frame 34 includes a slot 38 to receive the lock bar 22. A pair of holes 40 in the frame 34 receive the studs 30 and thereby prevent relative movement between the restraint member 32 and the lock bar 22.

In use, the lock bar 22 with the mounted restraint member 32 is positioned over the patient. The end members 24 of the lock bar 22 are snap fit onto the desired pair of nodules 20 in the table top 12 so as to secure the lock bar 22 to the table top 12. The spherical shape of the nodules 20 and recesses 26 provide a self-centering snap fit of the lock bar 22 to the table top 12. The bar 22 has a tapered surface 42 to facilitate placement of the end member 24 onto the nodules 20.

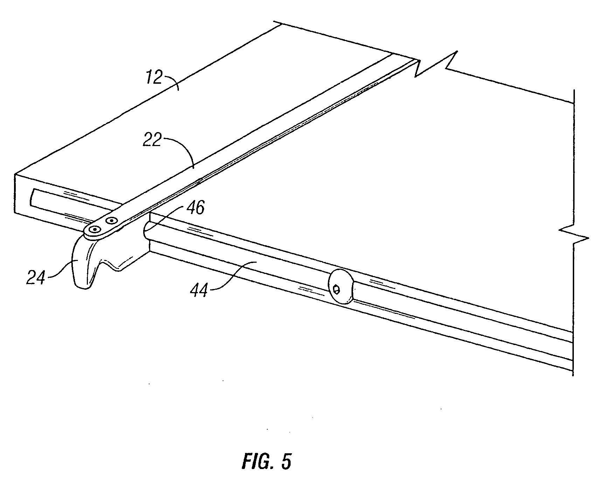

A second embodiment of the invention is shown in Figure 5. Rather than separate, spaced nodules 20, a continuous raised male bead 44 is provided on the edges of table top 12. A corresponding groove 46 is provided in the end member 24 of the lock bar 22 to matingly fit onto the bead 44.

Whereas the invention has been shown and described in connection with the preferred embodiment thereof, it will be understood that any modifications, substitutions, and additions may be made which are within the intended broad scope of the following claims. From the foregoing, it can be seen that the present invention accomplishes at least all of the stated objectives.

## Claims

1. A device for positioning a patient for treatment comprising: a table for supporting the patient, and having opposite sides with indexing nodules extending outwardly along each side; a lock bar having opposite ends with a recess on each end to retentively engage opposing pairs of the nodules on each side of the table; and a patient restraint member mated to the lock bar.

2. The device of claim 1 wherein the lock bar snap fits onto the nodules.

3. The device of claim 1 wherein the nodules and the recesses have mating shapes.

4. The device of claim 1 wherein the nodules are on a strip secured to the table on each side.

5. The device of claim 1 wherein the lock bar includes pins and the restraint member includes apertures to receive the pins.

6. The device of claim1 wherein the restraint member includes a slot for receiving the lock bar.

7. The device of claim 6 wherein the lock bar includes pins and the restraint member includes apertures to receive the pins.

8. A device for positioning a patient on a treatment table, comprising: a table top with opposite side edges and a male projection along each edge; a bar having opposite ends a female recess in each end; a patient restraint member coupled with the bar; and the female recesses on the ends of the bar being adapted to matingly receive the male projections on opposite edges of the table to selectively position the patient on the table.

9. The device of claim 8 wherein the male extensions are on a strip of material secured to each side edge of the table.

10. The device of claim 8 wherein the bar snap fits onto the table.

11. The device of claim 8 wherein the restraint member has a slot to receive the bar.

12. The device of claim 8 wherein the restraint member has apertures and the bar has pins adapted to extend into the bar apertures.

13. A method of restraining a patient on a table, comprising: positioning the patient on the table; mounting a restraint member on a lock bar; extending the restraint member over the patient; and snap fitting opposite ends of the bar onto male indexing nodules along each side of the table.
